# FASCICULE DE BREVET EUROPEEN

(11) **EP 2 705 050 B1**
(45) Date de publication et mention de la délivrance du brevet: **24.02.2016**
(21) Numéro de dépôt: 12725474.6
(22) Date de dépôt: 27.04.2012
(51) Int. Cl.: C07K 7/06, A61K 38/08, C07K 14/47

(54) **NOUVEAUX PEPTIDES MODULATEURS DE LA PROTEINE TRF2 ET COMPOSITIONS LES COMPRENANT**
NEUE PEPTIDE ALS MODULATOREN DES PROTEINS TRF2 UND DIESE ENTHALTENDE ZUSAMMENSETZUNGEN
NOVEL PEPTIDES THAT ARE MODULATORS OF THE PROTEIN TRF2 AND COMPOSITIONS CONTAINING SAME

(30) Priorité: 02.05.2011 FR 1101348
(43) Date de publication de la demande: 12.03.2014
(73) Titulaire: Isp Investments Inc., Wilmington, DE 19805 (US)
(72) Inventeur: DAL FARRA, Claude, Kerhonkson, New York 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2012/000166
(87) Numéro de publication internationale: WO 2012/153015

(56) Documents cités:
- EP-A2- 1 721 972
- WO-A1-99/15662
- WO-A2-01/44266
- BILAUD T ET AL: "THE TELOBOX, A MYB-RELATED TELOMERIC DNA BINDING MOTIF FOUND IN PROTEINS FROM YEAST, PLANTS AND HUMAN", NUCLEIC ACIDS RESEARCH, OXFORD UNIVERSITY PRESS, SURREY, GB, vol. 24, no. 7, 1 avril 1996 (1996-04-01), pages 1294-1303, XP002019946, ISSN: 0305-1048, DOI: 10.1093/NAR/24.7.1294

### SEQUENCE LISTING

<110> ISP Investments Inc
<120> NOUVEAUX PEPTIDES MODULATEURS DE LA PROTEINE TRF-2 ET COMPOSITIONS LES COMPRENANT
<130> Bv PCT 11-153
<150> FR1101348
   <151> 2011-05-02
<160> 6
<170> PatentIn version 3.5
<210> 1
   <211> 9
   <212> PRT
   <213> Artificial
<220>
   <223> Synthetic peptide
<220>
   <221> MOD_RES
   <222> (9)..(9)
   <223> AMIDATION
<400> 1
   Asn Thr Lys Lys Gln Lys Trp Thr Asn
   1               5
<210> 2
   <211> 5
   <212> PRT
   <213> Artificial
<220>
   <223> Synthetic peptide
<220>
   <221> MOD_RES
   <222> (5)..(5)
   <223> AMIDATION
<400> 2
   Lys Lys Gln Lys Trp
   1               5
<210> 3
   <211> 11
   <212> PRT
   <213> Artificial
<220>
   <223> synthetic peptide
<400> 3
   Gly Gly Leu Lys Lys Gln Lys Trp Asn Leu Tyr
   1 5 10
<210> 4
   <211> 7
   <212> PRT
   <213> Artificial
<220>
   <223> Synthetic peptide
<220>
   <221> MOD_RES
   <222> (7)..(7)
   <223> AMIDATION
<400> 4
   Ala Met Lys Lys Gln Lys Trp
   1 5
<210> 5
   <211> 9
   <212> PRT
   <213> Artificial
<220>
   <223> Synthetic peptide
<400> 5
   Ala Leu Ser Lys Lys Gln Lys Trp Gln
   1 5
<210> 6
   <211> 7
   <212> PRT
   <213> Artificial
<220>
   <223> synthetic peptide
<220>
   <221> MOD_RES
   <222> (7)..(7)
   <223> AMIDATION
<400> 6
   Cys Lys Lys Gln Lys Trp Ser
   1 5

## Description

La présente invention se situe dans le domaine de la cosmétique. Elle se rapporte à des composés peptidiques de formule générale (I) suivante :

R₁-(AA)ₙ-X₁-X₂-X₃-Lys-Lys-Gln-Lys-Trp-X₄-(AA)ₚ-R₂ (I)

en tant que composés modulateurs de la protéine TRF2, ayant ainsi une action protectrice des télomères et une action préventive sur les cassures doubles brins d'ADN. Lesdits composés peptidiques inclus dans une composition cosmétique sont destinés à prévenir et/ou retarder et/ou traiter les signes cutanés du vieillissement.

La sénescence est un processus biologique qui atteint toutes les cellules « normalement » constituées d'un être humain. Celle-ci se traduit d'un point de vue physique par un vieillissement global du corps humain, que ce soit les cellules de la peau, des cheveux, des organes...

Il a été démontré scientifiquement que la sénescence est causée notamment par le raccourcissement des télomères (sénescence réplicative) ou par l'exposition aiguë ou chronique à des signaux de stress physiologiques comme par exemple les stress oxydatifs. Un des moyens explorés afin de limiter la sénescence des cellules et par conséquent limiter les manifestations du vieillissement chez l'être humain, consiste à agir sur le raccourcissement des télomères et/ou limiter l'action des stress oxydatifs sur l'ADN.

Les télomères sont formés de répétitions de la séquence TTAGGG et de protéines spécifiques. Les télomères sont situés aux extrémités des chromosomes et assurent la stabilité de ceux-ci. Parmi les protéines qui s'associent aux télomères, on trouve les protéines TRF1 et TRF2 (TRF pour telomere-binding factor) qui se fixent directement sur l'ADN télomérique double brin, ou encore la protéine POT1 qui elle, se lie, à l'extrémité 3' simple brin. Au cours des divisions cellulaires, des motifs TTAGGG sont perdus en cours de route lors de la réplication. Cette perte de télomères est partiellement compensée par une transcriptase inverse, la télomérase, qui synthétise *de novo* des répétitions télomèriques sur des extrémités de télomères préexistants, assurant ainsi une longueur optimum. Le problème qui se pose, est que l'activité de cette télomérase est extrêmement faible dans les cellules somatiques, ceci conduisant à un raccourcissement télomérique à chaque réplication chromosomique. Une des stratégies utilisée à l'heure actuelle pour limiter le vieillissement de la peau ainsi que ses manifestations sur celle-ci, consiste à augmenter l'activité télomérase des cellules de la peau. Cependant, on s'expose ainsi à une immortalisation desdites cellules, ce qui n'est pas souhaitable car les cellules immortalisées peuvent s'apparenter à des cellules cancéreuses.

La Demanderesse a exploré une autre piste pour tenter de retarder la sénescence cellulaire, en modulant la quantité de protéines associées aux télomères, et plus particulièrement la protéine TRF2. Ainsi, la Demanderesse a mis au point des composés peptidiques de formule générale (I) suivante :

R₁-(AA)ₙ-X₁-X₂-X₃-Lys-Lys-Gln-Lys-Trp-X₄-(AA)ₚ-R₂ (I)

Lesdits composés sont capables de prévenir et/ou retarder et/ou traiter les signes cutanés du vieillissement par modulation de la quantité des protéines associées aux télomères (TRFs). Ils sont en outre utiles afin de prévenir la survenue des cassures doubles brins au niveau de l'ADN des cellules de la peau lors de dommages grâce à la modulation de la quantité de protéines TRFs présentes dans la cellule.

Des composés modulateurs des protéines TRFs ou encore stabilisateurs des télomères ont été proposés auparavant comme exposés dans les demandes de brevet US20020076719, WO9836066, ou encore WO2004092395. Cependant, aucun des documents cités précédemment ne décrit ni ne divulgue des composés peptidiques tels qu'exposés dans la présente demande de brevet, ni l'utilisation cosmétique de tels composés en tant qu'agent luttant contre les signes cutanés du vieillissement.

La présente invention a donc pour premier objet un composé peptidique de formule générale suivante (I) :

R₁-(AA)ₙ-X₁-X₂-X₃-Lys-Lys-Gln-Lys-Trp-X₄-(AA)ₚ-R₂ (I)

selon la revendication 1

La présente invention a pour second objet une composition cosmétique comprenant, à titre de principe actif, ledit composé peptidique de formule (I). selon la revendication 1

En outre, la présente invention a pour troisième objet l'utilisation cosmétique d'une composition comprenant ledit composé peptidique de formule (I) pour prévenir et/ou retarder et/ou traiter les signes cutanés du vieillissement. En particulier, ladite composition cosmétique sera utile (i) pour moduler la quantité de protéines associées aux télomères (TRFs) et/ou retarder la sénescence dans les cellules de la peau, (ii) pour prévenir la survenue des cassures doubles brins au niveau de l'ADN des cellules de la peau lors de dommages, (iii) pour prévenir et/ou traiter les signes cutanés du photovieillissement de la peau, et enfin (iv) pour protéger la peau des agressions extérieures.

Enfin, la présente invention a pour quatrième objet un procédé de traitement cosmétique de la peau ou des phanères à traiter à l'aide de la composition comprenant ledit composé peptidique de formule (I), ou encore un procédé de traitement cosmétique à l'aide du peptide selon l'invention pour limiter la dégradation des télomères sans modifier la quantité et/ou l'activité de la télomérase.

Les composés peptidiques selon l'invention se caractérisent par le fait qu'ils :
- modulent la quantité de protéines associées aux télomères (TRFs) et en particulier la protéine TRF2 ;
- limitent l'augmentation et l'agrégation de la protéine du cytosquelette Vimentine dans les cellules ayant un phénotype de cellules âgées ;
- limitent l'apparition de dommages dans des biopsies de peaux soumises à du méthylglyoxal ; et
- permettent en conclusion de prévenir et/ou retarder et/ou traiter les signes de sénescence dans les cellules traitées.

Le premier objet de la présente invention se rapporte à un composé peptidique de formule générale (I) :

R₁-(AA)ₙ-X₁-X₂-X₃-Lys-Lys-Gln-Lys-Trp-X₄-(AA)ₚ-R₂ (I)

dans laquelle,
X₁ représente une asparagine, une glycine, une thréonine, une alanine ou aucun acide aminé,
X₂ représente une isoleucine, une leucine, une méthionine, une proline, ou aucun acide aminé,
X₃ représente une thréonine, une asparagine, une sérine ou aucun acide aminé,
X₄ représente une sérine, une cystéine, une thréonine, une asparagine, une glutamine ou aucun acide aminé,
AA représente un acide aminé quelconque, et n et p sont des nombres entiers compris entre 0 et 2,
R₁ représente la fonction amine primaire de l'acide aminé N-terminal, -NH2, dans laquelle un des deux atomes d'hydrogène peut être substitué ou non substitué, soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal,-OH, dans lequel l'atome d'hydrogène peut être substitué ou non substitué, soit par une chaîne alkyle de C₁ à C₃₀, soit par un groupement NH2, NHY ou NYY, dans lequel Y représente une chaîne alkyle de C₁ à C₄,
ladite séquence de formule générale (I) étant constituée de 5 à 13 résidus d'acides aminés.

Le terme « composé peptidique » ou « peptide » désigne un enchaînement de deux ou plusieurs acides aminés liés entre eux par des liaisons peptidiques ou par des liaisons peptidiques modifiées.

Par « composé peptidique » ou « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

Les acides aminés constituant le composé peptidique selon l'invention peuvent être sous configuration Lévogyre, c'est-à-dire L-, et/ou Dextrogyre, c'est-à-dire D-. Le peptide selon l'invention peut donc être sous forme L-, D- ou DL-.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine de la cosmétique. De préférence, on utilise pour protéger la fonction amine primaire de l'acide aminé N-terminal, une substitution par un groupement R₁ de type acyle possédant une chaîne alkyle de C₁ à C₃₀, saturée ou insaturée, pouvant être choisie parmi un groupement acétyle ou un groupement aromatique. De préférence, on utilise pour protéger la fonction carboxyle de l'acide aminé C-terminal, une substitution par un groupement R₂ de type chaîne alkyle de C₁ à C₃₀, ou un groupement NH₂, NHY ou NYY avec Y représentant une chaîne alkyle de C₁ à C₄.

Le peptide selon l'invention peut être protégé au niveau de l'extrémité N-terminale, C-terminale ou au niveau des deux extrémités.

Dans un premier mode de réalisation de l'invention, dans la formule générale (I), n et p sont égaux à zéro et la séquence de formule générale (I) est constituée de 5 à 9 résidus d'acides aminés. Ceci signifie donc que dans la formule générale (I) :
X₁ représente une asparagine, une glycine, une thréonine, une alanine ou aucun acide aminé,
X₂ représente une isoleucine, une leucine, méthionine, une proline, ou aucun acide aminé,
X₃ représente une thréonine, une asparagine, une sérine ou aucun acide aminé,
X₄ représente une sérine, une cystéine, une thréonine, une asparagine, une glutamine ou aucun acide aminé,
les nombres entiers n et p sont égaux à zéro,
R₁ représente la fonction amine primaire de l'acide aminé N-terminal, -NH2, dans laquelle un des deux atomes d'hydrogène peut être substitué ou non substitué, soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, - OH, dans lequel l'atome d'hydrogène peut être substitué ou non substitué, soit par une chaîne alkyle de C₁ à C₃₀, soit par un groupement NH2, NHY ou NYY, dans lequel Y représente une chaîne alkyle de C₁ à C₄,
ladite séquence de formule générale (I) étant constituée de 5 à 9 résidus d'acides aminés.

Dans un second mode de réalisation préféré, le composé peptidique correspond à une des formules suivantes :
(SEQ ID n°1) Asn-Thr-Lys-Lys-Gln-Lys-Trp-Thr-Asn-NH₂
(SEQ ID n°2) Lys-Lys-Gln-Lys-Trp-NH₂
(SEQ ID n°3) Gly-Gly-Leu-Lys-Lys-Gln-Lys-Trp-Asn-Leu-Tyr
(SEQ ID n°4) Ala-Met-Lys-Lys-Gln-Lys-Trp-NH₂
(SEQ ID n°5) Ala-Leu-Ser-Lys-Lys-Gln-Lys-Trp-Gln
(SEQ ID n°6) Cys-Lys-Lys-Gln-Lys-Trp-Ser-NH₂

L'invention concerne aussi des formes homologues de ces séquences. Le terme « homologue » désigne, selon l'invention, toute séquence peptidique identique à au moins 50 %, ou de préférence au moins 80 %, et encore plus préférentiellement à au moins 90 % de ladite séquence peptidique, choisie parmi les séquences SEQ ID n° 1 à SEQ ID n° 6. Par « séquence peptidique identique à au moins X % », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par le logiciel informatique BLAST P disponible sur le site NCBI.

Le terme « homologue » peut également désigner un peptide qui diffère de la séquence d'un peptide de séquence SEQ ID n° 1 à SEQ ID n° 6 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.

Le peptide de formule générale (I) selon l'invention peut être obtenu soit par synthèse chimique classique (en phase solide ou en phase homogène liquide), soit par synthèse enzymatique (Kullmann et al., J. Biol. Chem. 1980 ; 225 : 8234) à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Enfin, le principe actif peut être un peptide unique, un mélange de peptides ou de dérivés peptidiques et/ou constitué de dérivés d'acides aminés.

Selon un mode de réalisation avantageux de l'invention, le composé peptidique selon l'invention est solubilisé dans un ou plusieurs solvants physiologiquement adaptés, classiquement utilisés par l'homme du métier, comme l'eau, le glycérol, l'éthanol, le propanediol, le butylène glycol, le dipropylène glycol, les diglycols éthoxylés ou propoxylés, les polyols cycliques, ou tout mélange de ces solvants.

Selon encore un autre mode de réalisation avantageux de l'invention, le composé peptidique selon l'invention est solubilisé dans un vecteur cosmétique comme les liposomes, ou adsorbé sur des polymères organiques poudreux, des supports minéraux comme les talcs et bentonites, et plus généralement solubilisé dans, ou fixé sur, tout vecteur physiologiquement adapté.

Le second objet de la présente invention se rapporte à une composition cosmétique comprenant à titre de principe actif ledit composé peptidique de formule générale (I).
De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Les compositions destinées à être appliquées sur la peau peuvent se présenter sous forme de crème, émulsion huile-dans-eau, ou eau-dans-huile ou émulsion multiple, solution, suspension, microémulsion, gel aqueux ou anhydre, sérum, ou encore de dispersion de vésicules, de patch, de spray, d'onguent, de pommade, de lotion, de colloïde, de lait, de lotion, de stick ou encore de poudre, tous adaptés à une application sur la peau, les lèvres et/ou les phanères.

Préférentiellement, ledit composé peptidique est présent dans la composition à une concentration comprise entre 0,1 et 500 ppm, et préférentiellement à une concentration comprise entre 1 et 150 ppm.

Encore plus préférentiellement, la composition selon l'invention contient, en outre, au moins un autre principe actif. On peut citer, de manière non limitative, les ingrédients suivants : des hydrolysats peptidiques issus de végétaux, d'autres composés peptidiques, des filtres solaires, des agents anti-radicalaires, ou encore des agents antirides. On peut citer également par exemple, la vitamine C et ses dérivés, les vitamines du groupe B, la DHEA (dihydroépiandrostérone), les phytostérols, l'acide salicylique et ses dérivés, les rétinoïdes, les flavonoïdes, les aminés de sucres, les azoles, les sels métalliques, ou encore les polymères.

La composition peut en outre comprendre des agents cicatrisants, antiâge, antirides, apaisants, des agents hydratants, anti-inflammatoires, des agents modulant la différenciation, la pigmentation ou la dépigmentation cutanée etc.

Dans un mode plus particulier de réalisation, la composition selon l'invention comprendra, outre le composé peptidique de formule (I) :
- un (ou plusieurs) composé activateur du cytochrome c, et/ou ;
- un (ou plusieurs) composé activateur des aquaporines et/ou ;
- un (ou plusieurs) composé activateur des sirtuines et/ou ;
- un (ou plusieurs) composé qui augmente l'adhésion cellulaire et/ou ;
- un (ou plusieurs) composé qui augmente la production de protéines matricielles type collagène, laminine etc. ;
- un (ou plusieurs) composé modulateur des protéines hsp ;
- un (ou plusieurs) composé qui augmente l'énergie cellulaire ;
- un (ou plusieurs) composé améliorant la fonction barrière de la peau ;
- un (ou plusieurs) composé améliorant la synchronisation des cellules comme par exemple des agents modulateurs des protéines Clock, Per1 ou Bmal ;
- un(ou plusieurs) composé protecteur de la mitochondrie.

Lesdits composés ci-dessus peuvent être naturels, comme des hydrolysats peptidiques ou non peptidiques de végétaux, ou encore d'origine synthétique, comme des composés peptidiques.

En outre, des additifs tels que des solvants, des diluants, des colorants, des filtres solaires, des agents auto-bronzants, des pigments, des charges, des conservateurs, des absorbeurs d'odeurs, des agents épaississants, émulsifiants, humectants, émollients, des parfums, des antioxydants, des agents filmogènes, chélatants, séquestrants, conditionnants peuvent être ajoutés à la composition.

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent être, par exemple, compris entre 0,01 et 20 % du poids total de la composition. Lorsque la composition de l'invention est une émulsion, la phase grasse peut représenter de 5 à 80 % en poids, et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids par rapport au poids total de la composition.

Un troisième objet de l'invention se rapporte à l'utilisation cosmétique d'une composition comprenant ledit composé peptidique dans un milieu cosmétiquement acceptable pour prévenir et/ou retarder et/ou traiter les signes cutanés du vieillissement. Les « signes cutanés du vieillissement » incluent, mais ne se limitent pas à, toutes les manifestations visibles sur la peau causées par le vieillissement. On entend, en particulier, les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, on entend aussi par « signes cutanés du vieillissement », les pores élargis, les imperfections, la décoloration, les taches de vieillesse, les kératoses, la perte de collagène, et autres changements du derme et de l'épiderme, mais également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme.

Dans un premier mode de réalisation de l'invention, ladite composition cosmétique permet de moduler la quantité de protéines associées aux télomères (TRFs) et/ou retarder la sénescence dans les cellules de la peau. Par « moduler la quantité de protéines associées aux télomères (TRFs) » on entend augmenter ou encore diminuer la quantité de protéines TRFs présentent dans les cellules, soit par augmentation/diminution de la synthèse protéique de celles-ci (par modulation directe ou indirecte de l'expression génique), soit par d'autres processus biologiques tels que la stabilisation/déstabilisation des transcrits d'ARN messager. Préférentiellement, les protéines TRFs sont représentées par les protéines TRF2 et/ou POT1.

Dans un second mode de réalisation préférentiel de l'invention, la composition cosmétique est utilisée afin de prévenir la survenue des cassures doubles brins au niveau de l'ADN des cellules de la peau lors de dommages. En effet, lors de dommages infligés aux cellules de la peau, notamment les dommages dus aux rayonnements UV ou encore les stress oxydatifs, l'ADN desdites cellules est susceptible de subir des dommages se manifestant par des cassures doubles brins. Les composés peptidiques selon l'invention ont démontrés leur action protectrice de l'ADN vis-à-vis de ce genre de dommages.

Une autre utilisation du peptide selon l'invention consiste à prévenir et/ou traiter les signes cutanés du photo-vieillissement de la peau. Par « photo-vieillissement », on entend le vieillissement prématuré de la peau causé par l'exposition prolongée et cumulative au soleil.

Le peptide selon l'invention peut également être utilisé afin de protéger la peau des agressions extérieures. Par « agressions extérieures », on entend les agressions que peut produire l'environnement. A titre d'exemple, on peut citer des agressions telles que la pollution, les rayonnements UV, les agressions provoquant des stress oxydatifs, ou encore les produits à caractère irritant tels que les tensioactifs, les conservateurs ou les parfums, les agressions mécaniques, telles que les abrasions, le rasage ou l'épilation. Cependant, préférentiellement, les agressions extérieures sont principalement constituées par les rayonnements UV, et en particulier les UVB et les agressions induisant des stress oxydatifs.

Enfin, un dernier objet de la présente invention se rapporte à un procédé de traitement cosmétique caractérisé par l'application matin et soir sur la peau, d'une composition comprenant une quantité efficace de composé peptidique tel que défini précédemment, pour prévenir et/ou traiter les signes cutanés du vieillissement. Dans un autre mode de réalisation de la présente invention, il s'agit de mettre en place un procédé de traitement cosmétique de la peau à l'aide d'une composition selon l'invention, afin de limiter la dégradation des télomères sans modifier la quantité et/ou l'activité de la télomérase. En effet, le peptide selon l'invention permet de stabiliser et/ou limiter la dégradation des télomères sans pour cela intervenir au niveau de l'activité et/ou de la quantité de télomérase, ce qui est important car il n'y aura ainsi aucun risque d'immortalisation des cellules.

Les exemples suivants décrivent et démontrent l'efficacité de composés peptidiques tels que décrits selon l'invention mais ne doivent pas être interprétés comme une limitation de la présente invention.

### Exemple 1: Etude de l'effet du peptide SEQ ID n°2 sur des fibroblastes vieillis par sénescence réplicative

Des fibroblastes humains sont mis en culture dans un milieu spécifique et maintenus en culture en traitement long (pendant plus de 17 passages), à l'aide d'une application quotidienne du peptide SEQ ID n°2 à une concentration de 1 % ou de 3 %. Une expérience de détection par immunofluorescence de la vimentine est effectuée sur les cellules aux passages de culture 6 et 17. Les cellules sont ensuite lavées avec du PBS, fixées au formaldéhyde à 3,7 % pendant 10 minutes, perméabilisées à l'aide de 0,2 % de Triton X-100 pendant 10 minutes (Fisher Chemical) et incubées avec 1 % de BSA (Euromedex) pendant 15 minutes. Un anticorps anti-vimentine (Tebu Santa Cruz) est par la suite ajouté et incubé à une dilution de 1/200^{ième} pendant 2 h à température ambiante. Après lavage au PBS, un anticorps anti-souris Donkey IgG, conjugué à un marqueur Alexa Fluor® 488 sont ajoutés à une dilution de 1/1000^{ième} et laissés en incubation pendant une heure à température ambiante. Enfin, les coupes sont montés au Fluoromount G (Electron Microscopy Science) et examinées à l'aide d'un microscope (Nikon Eclipse 80i, grossissement 40x).

### Résultats/ Conclusions :

On constate que les fibroblastes vieillis traités à l'aide du peptide SEQ ID n°2 expriment moins de vimentine que les fibroblastes vieillis non traités. Or, l'augmentation et l'agrégation de la vimentine est associée aux modifications du cytosquelette qui accompagnent le phénomène de vieillissement. On peut donc conclure que le traitement à l'aide du composé peptidique SEQ ID n°2 selon l'invention a permis de limiter l'augmentation et l'agrégation de la vimentine dus au vieillissement des fibroblastes.

### Exemple 2 : Etude de l'effet du peptide SEQ ID n°4 sur un modèle de biopsies rendues sénescentes in vitro à l'aide de méthyglyoxal

Des biopsies de peaux humaines sont rendues sénescentes artificiellement *in vitro,* grâce à un traitement à l'aide de méthylglyoxal (MGO). Pour cela, des punch de 6 mm de biopsies de peaux humaines sont mises à incuber à l'interface air-liquide dans un milieu de culture spécifique. Elles sont ensuite traitées avec 5 mM ou 10 mM de MGO (Sigma) déposés à la surface des biopsies et dans le milieu de culture. Les biopsies sont par la suite traitées avec soit :
- condition 1 : 20 µL de PBS IX, soit
- condition 2 : 20 µL de peptide SEQ ID n°4 à 1 %, soit
- condition 3 : 20 µL de peptide SEQ ID n° 4 à 3 %.

Les biopsies sont fixées et incluses dans de la paraffine, et sont ensuite coupées en sections de 4 µm à l'aide d'un microtome. Un immunomarquage hématoxyline/éosine (H&E) est réalisé sur les sections découpées précédemment et leur morphologie et structure sont étudiées par observation microscopique (à l'aide d'un microscope Nikon Eclipse E600, objectif 40x).

### Résultats/Conclusions :

Dans la condition contrôle 1, c'est-à-dire sans ajout de peptide, on constate que le MGO a provoqué des dommages importants au niveau des biopsies de peaux, plus exactement au niveau de la structure de celles-ci. Les dommages provoqués sont dose-dépendant puisqu'on observe plus de dommages avec la quantité de 10 µM de MGO.

Lorsque les biopsies sont traitées à l'aide du composé peptidique SEQ ID n°4, on constate que les dommages infligés aux structures desdites biopsies sont beaucoup moins importants que dans les conditions contrôle. On constate que l'effet protecteur du peptide est dose-dépendant puisqu'on observe encore moins de dommages avec la dose de 3 % qu'avec la dose de 1%.

On peut ainsi conclure que le peptide SEQ ID n°4 a eu un effet protecteur des structures cellulaires lorsque celles-ci sont soumises à des stress conduisant à des dommages importants et une sénescence précoce.

### Exemple 3 : Etude de l'expression de la protéine TRF2 par siRNA dans des fibroblastes humains traités à l'aide du peptide SEO ID n°2

Afin de quantifier l'efficacité d'un composé peptidique selon l'invention, sur la surexpression de TRF2 dans une population de fibroblastes humains, le gène codant pour TRF2 a été « éteint » par utilisation de la technique du siRNA.

### Protocole :

Des cellules fibroblastes sont mises en culture en plaque 6 puits jusqu'à une confluence de 60 %. Le milieu de culture est renouvelé avec ajout du peptide SEQ ID n°2 à 1 % dans les conditions décrites ci-dessous. Ensuite, 100 µL d'un mélange préalablement réalisé contenant le siRNA de TRF2 à 10 nM final et l'agent transfectant sont rajoutés délicatement au goutte à goutte, puit par puit. La plaque de culture cellulaire est incubée à 37°C et à 5 % en CO₂ pendant 72 h. Le milieu de culture est renouvelé tous les 2 jours. Quatre conditions ont été mises en place :
- condition 1 : contrôle sans siRNA et sans actif
- condition 2 : cellules transfectées avec le siRNA, sans actif
- condition 3 : cellules non transfectées mais traitées avec l'actif
- condition 4 : cellules transfectées avec le siRNA et traitées avec l'actif

La quantification de l'expression de TRF2 est observée par la technique classique d'immunotransfert (Western Blot) réalisée à l'aide d'un anticorps anti-TRF2 et selon un protocole classique. Afin d'analyser la compensation apportée par le peptide dans les fibroblastes ayant été transfectés avec le siRNA, la comparaison sera faite par rapport à des fibroblastes non traités et dont le gène n'a pas été éteint par siRNA.

### Résultats/Conclusions :

Entre les conditions 1 et 3, on constate que l'ajout du peptide a entraîné une augmentation de l'expression de la protéine TRF2 de 17 % par rapport au contrôle. Entre les conditions 1 et 2, on constate bien l'effet du siRNA sur l'expression de la protéine TRF2 : en effet, celle-ci est en baisse de 26 %. Par contre, l'ajout de l'actif aux cellules transfectées avec le siRNA permet de restaurer l'expression de TRF2, et la baisse due à la présence de siRNA n'est plus que de 18 % par rapport à la condition contrôle.

En conclusion, l'actif selon l'invention a permis de compenser la diminution de l'expression de la protéine TRF2 (diminution induite par le siRNA spécifique) dans les fibroblastes traités.

### Exemple 4 : Composition d'une crème visage anti-âge

| ***Noms commerciaux*** | ***Noms INCI*** | **% *massique*** |
|---|---|---|
| ***Phase A*** | | |
| Montanov 68 | Cetearyl Alcohol (and) Cetearyl Glucoside | 6,00 |
| Squalane | Squalane | 3,00 |
| ISP Bonjour Refined shea butter | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Si-tecDM350 | Dimethicone | 1,50 |
| BHT | BHT | 0,01 |
| Coenzyme Q 10 | Ubiquinone | 0,10 |

| ***Phase B*** | | |
|---|---|---|
| Huile d'Avocat | Persea Gratissima (Avocado) Oil | 1,25 |
| Optiphen | Phenoxyethanol (and) Caprylyl Clycol | 1 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°5 | | 3 mg/kg |

| ***Noms commerciaux*** | ***Noms INCI*** | **% *massique*** |
|---|---|---|
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase Ajuste avant d'émulsionner A dans C. A environ 45°C, le carbomer est neutralisé par addition de la phase D. A environ 30°C, la phase E est ensuite additionnée sous légère agitation et le refroidissement est poursuivi jusqu'à 25°C. La phase F est alors additionnée si souhaité.

## Revendications

1. Composé peptidique de formule générale suivante (I) :
R₁-(AA)ₙ-X₁-X₂-X₃-Lys-Lys-Gln-Lys-Trp-X₄-(AA)ₚ-R₂
dans laquelle,
X₁ représente une asparagine, une glycine, une thréonine, une alanine ou aucun acide aminé,
X₂ représente une isoleucine, une leucine, une méthionine, une proline, ou aucun acide aminé,
X₃ représente une thréonine, une asparagine, une sérine ou aucun acide aminé,
X₄ représente une sérine, une cystéine, une thréonine, une asparagine, une glutamine ou aucun acide aminé,
AA représente un acide aminé quelconque, et n et p sont des nombres entiers compris entre 0 et 2,
R₁ représente la fonction amine primaire de l'acide aminé N-terminal, -NH2, dans laquelle un des deux atomes d'hydrogène peut être substitué ou non substitué, soit par une chaîne alkyle de C₁ à C₃₀ saturée ou insaturée de type acétyle, soit par un groupement aromatique de type benzoyle, tosyle ou benzyloxycarbonyle,
R₂ représente le groupe hydroxyle de la fonction carboxyle de l'acide aminé C-terminal, -OH, dans lequel l'atome d'hydrogène peut être substitué ou non substitué, soit par une chaîne alkyle de C₁ à C30, soit par un groupement NH2, NHY ou NYY, dans lequel Y représente une chaîne alkyle de C₁ à C₄,
ladite séquence de formule générale (I) étant constituée de 5 à 13 résidus d'acides aminés, **caractérisé en ce qu'**il correspond à une des formules suivantes :
(SEQ ID n°1) Asn-Thr-Lys-Lys-Gln-Lys-Trp-Thr-Asn-NH₂;
(SEQ ID n°2) Lys-Lys-Gln-Lys-Trp-NH₂;
(SEQ ID n°3) Gly-Gly-Leu-Lys-Lys-Gln-Lys-Trp-Asn-Leu-Tyr;
(SEQ ID n°4) Ala-Met-Lys-Lys-Gln-Lys-Trp-NH₂;
(SEQ ID n°5) Ala-Leu-Ser-Lys-Lys-Gln-Lys-Trp-Gln;
(SEQ ID n°6) Cys-Lys-Lys-Gln-Lys-Trp-Ser-NH₂.

2. Composé peptidique selon la revendication 1, **caractérisé en ce qu'**il est solubilisé dans un ou plusieurs solvants physiologiquement adaptés choisi parmi l'eau, le glycérol, l'éthanol, le propanediol, le butylène glycol, le dipropylène glycol, les diglycols éthoxylés ou propoxylés, les polyols cycliques, ou tout mélange de ces solvants.

3. Composition cosmétique comprenant à titre de principe actif ledit composé peptidique selon l'une des revendications 1 ou 2 et un milieu cosmétiquement acceptable.

4. Composition selon la revendication 3, **caractérisée en ce qu'**elle se présente sous une forme adaptée à l'application par voie topique choisie parmi une crème, émulsion huile- dans-eau, ou eau-dans-huile ou émulsion multiple, solution, suspension, microémulsion, gel aqueux ou anhydre, sérum, dispersion de vésicules, patch, spray, onguent, pommade, lotion, colloïde, lait, stick ou encore une poudre.

5. Composition selon l'une des revendications 3 ou 4, **caractérisée en ce que** ledit composé peptidique est présent dans la composition à une concentration comprise entre 0,1 et 500 ppm.

6. Composition selon la revendication 5, **caractérisée en ce que** ledit composé peptidique est présent dans la composition à une concentration comprise entre 1 et 150 ppm.

7. Composition selon l'une quelconque des revendications 3 à 6, **caractérisée en ce qu'**elle contient, en outre, au moins un autre principe actif choisi parmi des hydrolysats peptidiques issus de végétaux, d'autres composés peptidiques, des filtres solaires, des agents anti-radicalaires, ou encore des agents antirides.

8. Utilisation cosmétique d'une composition selon l'une des revendications 3 à 7 pour prévenir et/ou retarder et/ou traiter les signes cutanés du vieillissement.

9. Utilisation cosmétique selon la revendication 8 pour moduler la quantité de protéines associées aux télomères (TRFs) et/ou retarder la sénescence dans les cellules de la peau.

10. Utilisation cosmétique selon la revendication 9, **caractérisée en ce que** les protéines associées aux télomères sont représentées par TRF2 et/ou POT1.

11. Utilisation cosmétique selon la revendication 8 pour prévenir la survenue des cassures doubles brins au niveau de l'ADN des cellules de la peau lors de dommages.

12. Utilisation cosmétique selon la revendication 8, **caractérisée en ce que** les signes cutanés du vieillissement sont les rides, les rides profondes et grossières, les ridules, les crevasses, le relâchement des tissus cutanés et sous-cutanés, la perte de l'élasticité cutanée et l'atonie, la perte de fermeté, et de tonicité, et l'atrophie dermique.

13. Utilisation cosmétique selon la revendication 8 pour prévenir et/ou traiter les signes cutanés du photovieillissement de la peau.

14. Utilisation cosmétique selon la revendication 8 pour protéger la peau des agressions extérieures.

15. Utilisation cosmétique selon la revendication 14, **caractérisée en ce que** les agressions extérieures sont les rayonnements UV, ou encore les stress oxydatifs.

16. Procédé de traitement cosmétique **caractérisé en ce que** l'on applique, matin et soir sur la peau, une composition comprenant une quantité efficace de composé peptidique tel que défini dans l'une des revendications 1 ou 2, pour prévenir et/ou traiter les signes cutanés du vieillissement.

17. Procédé de traitement cosmétique **caractérisé en ce que** l'on applique de manière régulière, une composition comprenant une quantité efficace de composé peptidique tel que défini dans l'une des revendications 1 ou 2, pour limiter la dégradation des télomères sans modifier la quantité et/ou l'activité de la télomérase.

## Patentansprüche

1. Peptidverbindung der folgenden allgemeinen Formel (I):
R₁-(AA)ₙ-X₁-X₂-X₃-Lys-Lys-Gln-Lys-Trp-X₄-(AA)ₚ-R₂,
in der
X₁ für ein Asparagin, ein Glycin, ein Threonin, ein Alanin oder keine Aminosäure steht,
X₂ für ein Isoleucin, ein Leucin, ein Methionin, ein Prolin oder keine Aminosäure steht,
X₃ für ein Threonin, ein Asparagin, ein Serin oder keine Aminosäure steht,
X₄ für ein Serin, ein Cystein, ein Threonin, ein Asparagin, ein Glutamin oder keine Aminosäure steht,
AA für eine beliebige Aminosäure steht und n und p ganze Zahlen zwischen 0 und 2 sind,
R₁ für die primäre Aminofunktion der N-terminalen Aminosäure, -NH₂, steht, in der eines der zwei Wasserstoffatome unsubstituiert oder entweder durch eine gesättigte oder ungesättigte C₁- bis C₃₀-Alkylkette des Acetyltyps oder durch eine aromatische Gruppe des Benzoyltyps, des Tosyltyps oder des Benzyloxycarbonyltyps substituiert sein kann,
R₂ für die Hydroxylgruppe der Carboxylfunktion der C-terminalen Aminosäure, -OH, steht, in der das Wasserstoffatom unsubstituiert oder entweder durch eine C₁- bis C₃₀-Alkylkette oder durch eine NH₂-, NHY- oder NYY-Gruppe, in der Y für eine C₁- bis C₄-Alkylkette steht, substituiert sein kann,
wobei die besagte Sequenz der allgemeinen Formel (I) aus 5 bis 13 Aminosäureresten besteht, **dadurch gekennzeichnet, dass** sie einer der folgenden Formeln entspricht:
(SEQ ID Nr. 1) Asn-Thr-Lys-Lys-Gln-Lys-Trp-Thr-Asn-NH₂;
(SEQ ID Nr. 2) Lys-Lys-Gln-Lys-Trp-NH₂;
-(SEQ ID Nr. 3) Gly-Gly-Leu-Lys-Lys-Gln-Lys-Trp-Asn-Leu-Tyr;
(SEQ ID Nr. 4) Ala-Met-Lys-Lys-Gln-Lys-Trp-NH₂;
(SEQ ID Nr. 5) Ala-Leu-Ser-Lys-Lys-Gln-Lys-Trp-Gln;
(SEQ ID Nr. 6) Cys-Lys-Lys-Gln-Lys-Trp-Ser-NH₂.

2. Peptidverbindung nach Anspruch 1, **dadurch gekennzeichnet, dass** sie in einem oder mehreren physiologisch angepassten Lösungsmitteln solubilisiert wird, das bzw. die aus Wasser, Glycerin, Ethanol, Propandiol, Butylenglykol, Dipropylenglykol, ethoxylierten oder propoxylierten Diglykolen, cyclischen Polyolen oder jeglichem Gemisch dieser Lösungsmittel ausgewählt ist bzw. sind.

3. Kosmetische Zusammensetzung, die die besagte Peptidverbindung nach einem der Ansprüche 1 oder 2 als Wirkstoff und ein kosmetisch akzeptables Medium umfasst.

4. Zusammensetzung nach Anspruch 3, **dadurch gekennzeichnet, dass** sie in einer Form dargereicht wird, die auf eine topische Anwendung angepasst ist und die aus einer Creme, einer Öl-in-Wasser- oder Wasser-in-Öl-Emulsion oder multiplen Emulsion, einer Lösung, einer Suspension, einer Mikroemulsion, einem wässrigen oder wasserfreien Gel, einem Serum, einer Vesikeldispersion, einem Pflaster, einem Spray, einer Salbe, einer Pomade, einer Lotion, einem Kolloid, einer Milch, einem Stick oder auch einem Puder ausgewählt ist.

5. Zusammensetzung nach Anspruch 3 oder 4, **dadurch gekennzeichnet, dass** die besagte Peptidverbindung in der Zusammensetzung in einer Konzentration zwischen 0,1 und 500 ppm vorliegt.

6. Zusammensetzung nach Anspruch 5, **dadurch gekennzeichnet, dass** die besagte Peptidverbindung in der Zusammensetzung in einer Konzentration zwischen 1 und 150 ppm vorliegt.

7. Zusammensetzung nach einem der Ansprüche 3 bis 6, **dadurch gekennzeichnet, dass** sie außerdem mindestens einen anderen Wirkstoff enthält, der aus von Pflanzen stammenden Peptidhydrolysaten, anderen Peptidverbindungen, Sonnenschutzfiltern, Radikalfängern oder auch Antifaltenmitteln ausgewählt ist.

8. Kosmetische Verwendung einer Zusammensetzung nach einem der Ansprüche 3 bis 7 zum Verhindern und/oder Verzögern und/oder Behandeln von Zeichen der Alterung der Haut.

9. Kosmetische Verwendung nach Anspruch 8 zum Modulieren der Menge von Proteinen, die mit den Telomeren (TRF) assoziiert sind, und/oder zum Verzögern der Seneszenz in den Zellen der Haut.

10. Kosmetische Verwendung nach Anspruch 9, **dadurch gekennzeichnet, dass** die Proteine, die mit den Telomeren assoziiert sind, durch TRF2 und/oder POT1 dargestellt sind.

11. Kosmetische Verwendung nach Anspruch 8 zum Verhindern des Auftretens von Doppelstrangbrüchen auf DNA-Ebene von Zellen der Haut während Schädigungen.

12. Kosmetische Verwendung nach Anspruch 8, **dadurch gekennzeichnet, dass** es sich bei den Zeichen der Alterung der Haut um Falten, tiefe und grobe Falten, Fältchen, Risse, Erschlaffung der Haut- und Unterhautgewebe, Verlust der Hautelastizität und Atonie, Verlust der Festigkeit und des Tonus und dermale Atrophie handelt.

13. Kosmetische Verwendung nach Anspruch 8 zum Verhindern und/oder Behandeln der Zeichen der Lichtalterung der Haut.

14. Kosmetische Verwendung nach Anspruch 8 zum Schützen der Haut vor äußeren Einflüssen.

15. Kosmetische Verwendung nach Anspruch 14, **dadurch gekennzeichnet, dass** es sich bei den äußeren Einflüssen um UV-Strahlen oder auch oxidativen Stress handelt.

16. Verfahren zur kosmetischen Behandlung, **dadurch gekennzeichnet, dass** morgens und abends eine Zusammensetzung, die eine wirksame Menge der wie in einem der Ansprüche 1 oder 2 definierten Peptidverbindung umfasst, zum Verhindern und/oder Behandeln von Zeichen der Alterung der Haut auf die Haut angewendet wird.

17. Verfahren zur kosmetischen Behandlung, **dadurch gekennzeichnet, dass** regelmäßig eine Zusammensetzung, die eine wirksame Menge der wie in einem der Ansprüche 1 oder 2 definierten Peptidverbindung umfasst, zum Beschränken des Abbaus von Telomeren, ohne die Menge und/oder die Aktivität der Telomerase zu modifizieren, angewendet wird.

## Claims

1. Peptide compound of the following general formula (I):
R₁-(AA)ₙ-X₁-X₂-X₃-Lys-Lys-Gln-Lys-Trp-X₄ - (AA)ₚ -R₂
wherein,
X₁ represents an asparagine, a glycine, a threonine, an alanine or no amino acid,
X₂ represents an isoleucine, a leucine, a methionine, a proline, or no amino acid,
X₃ represents a threonine, an asparagine, a serine or no amino acid,
X₄ represents a serine, a cysteine, a threonine, an asparagine, a glutamine or no amino acid,
AA represents any amino acid, and n and p are integers between 0 and 2,
R₁ represents the primary amine function of the N-terminal amino acid, -NH2, wherein one of the two hydrogen atoms may or may not be substituted either by an acetyl-type saturated or an unsaturated C₁ to C₃₀ alkyl chain, or by an aromatic group of the benzoyl, tosyl or benzyloxycarbonyl type,
R₂ represents the hydroxyl group of the carboxyl function of the C-terminal amino acid, -OH, wherein the hydrogen atom may or may not be substituted either by a C₁ to C₃₀ alkyl chain, or an NH₂, NHY or NYY group, wherein Y represents a C₁ à C₄ alkyl chain,
said sequence of general formula (I) consisting of 5 to 13 amino acid residues, **characterized in that** it corresponds to one of the following formulas:
(SEQ ID no. 1) Asn - Thr - Lys - Lys - Gln - Lys - Trp-Thr - Asn - NH₂
(SEQ ID no. 2) Lys - Lys - Gln - Lys - Trp - NH₂ (SEQ ID no. 3) Gly - Gly - Leu - Lys - Lys - Gln - Lys-Trp - Asn - Leu - Tyr
(SEQ ID no. 4) Ala - Met - Lys - Lys - Gln - Lys - Trp - NH₂
(SEQ ID no. 5) Ala - Leu - Ser - Lys - Lys - Gln - Lys - Trp - Gln
(SEQ ID no. 6) Cys - Lys - Lys - Gln - Lys - Trp - Ser - NH₂

2. Peptide compound according to claim 1, **characterized in that** it is solubilized in one or more physiologically suitable solvents chosen from water, glycerol, ethanol, propanediol, butylene glycol, dipropylene glycol, ethoxylated or propoxylated diglycols, cyclic polyols or any mixture of these solvents.

3. Cosmetic composition including, as an active principle, said peptide compound according to one of claims 1 or 2 and a cosmetically acceptable medium.

4. Composition according to claim 3, **characterized in that** it is in a form suitable for topical application, chosen from a cream, an oil-in-water emulsion, or a water-in-oil or multiple emulsion, a solution, a suspension, a microemulsion, an aqueous or anhydrous gel, a serum or a vesicle dispersion, a patch, a spray, an ointment, a pomade, a lotion, a colloid, a milk, a stick or a powder.

5. Composition according to one of claims 3 or 4, **characterized in that** said peptide compound is present in the composition at a concentration of between 0.1 and 500 ppm.

6. Composition according to claim 5, **characterized in that** said peptide compound is present in the composition at a concentration of between 1 and 150 ppm.

7. Composition according to any one of claims 3 to 6, **characterized in that** it also contains at least one other active principle chosen from plant peptide hydrolysates, other peptide compounds, sunscreens, anti-free radical agents or anti-wrinkle agents.

8. Cosmetic use of a composition according to one of claims 3 to 7 for preventing and/or delaying and/or treating cutaneous signs of aging.

9. Cosmetic use according to claim 8 for modulating the quantity of telomere-associated proteins (TRFs) and/or delaying senescence in skin cells.

10. Cosmetic use according to claim 9, **characterized in that** the proteins associated with the telomeres are represented by TRF2 and/or POT1.

11. Cosmetic use according to claim 8 for preventing the occurrence of DNA double-strand breaks in skin cells upon damage.

12. Cosmetic use according to claim 8, **characterized in that** the cutaneous signs of aging are wrinkles, deep and rough wrinkles, fine lines, cracks, loosening of cutaneous and subcutaneous tissue, loss of cutaneous elasticity and atonia, loss of firmness and tone, and dermal atrophy.

13. Cosmetic use according to claim 8 for preventing and/or treating the cutaneous signs of photoaging of the skin.

14. Cosmetic use according to claim 8 for protecting the skin from external stresses.

15. Cosmetic use according to claim 14, **characterized in that** the external stresses are UV radiation or oxidative stress.

16. Cosmetic treatment method **characterized by** the application, in the morning and evening, on the skin, of a composition including an effective quantity of the peptide compound as defined in one of claims 1 or 2, for preventing and/or treating cutaneous signs of aging.

17. Cosmetic treatment method **characterized by** the regular application of a composition including an effective quantity of a peptide compound as defined in one of claims 1 or 2, for limiting the degradation of telomeres without modifying the quantity and/or the activity of telomerase.
